(19) Europäisches Patentamt
European Patent Office
Office européen des brevets

(11) Veröffentlichungsnummer: 0 253 259 A2

# (12) EUROPÄISCHE PATENTANMELDUNG

(21) Anmeldenummer: 87109696.2

(22) Anmeldetag: 06.07.87

(51) Int. Cl.4: **A01N 47/18**, A01N 47/22, C07D 333/38, C07D 333/62, C07D 333/78

(30) Priorität: 18.07.86 DE 3624304

(43) Veröffentlichungstag der Anmeldung: 20.01.88 Patentblatt 88/03

(84) Benannte Vertragsstaaten: BE CH DE FR GB IT LI NL

(71) Anmelder: BAYER AG
Konzernverwaltung RP Patentabteilung
D-5090 Leverkusen 1 Bayerwerk(DE)

(72) Erfinder: Lindel, Hans, Dr.
Carl-Duisberg-Strasse 321
D-5090 Leverkusen 1(DE)
Erfinder: Hallenbach, Werner, Dr.
Kleiststrasse 10
D-4018 Langenfeld(DE)
Erfinder: Brandes, Wilhelm, Dr.
Eichendorffstrasse 3
D-5653 Leichlingen(DE)
Erfinder: Reinecke, Paul, Dr.
Steinstrasse 8
D-5090 Leverkusen 3(DE)

## (54) Fungizide Verwendung von substituierten Thienylaminen.

(57) Verwendung von substituierten Thienylaminen der allgemeinen Formel

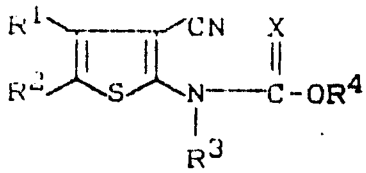

in welcher

X für Sauerstoff oder Schwefel steht,

$R^1$ und $R^2$ gleich oder verschieden sind und für Wasserstoff, Halogen, Nitro, Cyano, Alkoxy, Alkylthio, Halogenalkoxy, Halogenalkylthio oder für gegebenenfalls substituierte Reste aus der Reihe Alkyl, Aryl, Alkylcarbonyl oder Arylcarbonyl stehen, oder

$R^1$ und $R^2$ gemeinsam mit den angrenzenden beiden Kohlenstoffatomen für einen gegebenenfalls durch Alkyl substituierten gesättigten oder ungesättigten carbocyclischen Ring stehen,

$R^3$ für Wasserstoff oder Alkyl steht und

EP 0 253 259 A2

$R^4$ für gegebenenfalls substituiertes Alkyl, für Cycloalkyl, Alkenyl oder für gegebenenfalls substituiertes Aryl steht,

als Fungizide im Pflanzenschutz.

## Fungizide Verwendung von substituierten Thienylaminen

Die vorliegende Erfindung betrifft die Verwendung von substituierten Thienylaminen als Fungizide im Pflanzenschutz.

Thienylamine sind bereits bekannt. Sie finden Verwendung als Herbizide, Zwischenprodukte zur Farbstoffherstellung und Pharmazeutika (vergl. DE-OS 21 22 636, EP-OS 4 931, US-P 4 317 915, 4 447 624). Über ihre Verwendung als Fungizide ist nichts bekannt.

Es ist außerdem bekannt geworden, daß bestimmte Thienylharnstoffe, wie z. B. 1-(4,5-Dimethyl-3-ethoxycarbonyl-2-thienyl)-3-methylharnstoff, eine gute fungizide Wirksamkeit besitzen (vergl. z. B. US-PS 3 823 161). Weiterhin ist bekannt, daß Benzimidazolcarbamate, wie Benzimidazol-2-yl-carbaminsäuremethylester sehr breit wirksame Fungizide sind. Die Anwendung dieser Verbindungen wird jedoch durch Resistenzerscheinungen eingeschränkt.

Es wurde gefunden, daß die substituierten Thienylamine der allgemeinen Formel (I)

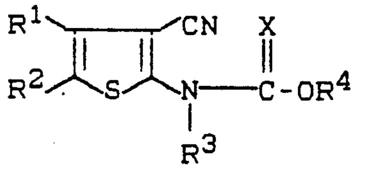

(I)

in welcher

X für Sauerstoff oder Schwefel steht,

$R^1$ und $R^2$ gleich oder verschieden sind und für Wasserstoff, Halogen, Nitro, Cyano, Alkoxy, Alkylthio, Halogenalkoxy, Halogenalkylthio oder für gegebenenfalls substituierte Reste aus der Reihe Alkyl, Aryl, Alkylcarbonyl oder Arylcarbonyl stehen, oder

$R^1$ und $R^2$ gemeinsam mit den angrenzenden beiden Kohlenstoffatomen für einen gegebenenfalls durch Alkyl substituierten gesättigten oder ungesättigten carbocyclischen Ring stehen,

$R^3$ für Wasserstoff oder Alkyl steht und

$R^4$ für gegebenenfalls substituiertes Alkyl, für Cycloalkyl, Alkenyl oder für gegebenenfalls substituiertes Aryl steht,

als Fungizide im Pflanzenschutz verwendet werden können.

Überraschenderweise zeigen die substituierten Thienylamine der allgemeinen Formel (I) eine erheblich höhere fungizide Wirkung als die aus dem Stand der Technik bekannte Verbindung 1-(4,5-Dimethyl-3-ethoxycarbonyl-2-thienyl)-3-methylharnstoff, welche konstitutionell und wirkungsmäßig eine ähnliche Verbindung ist (vergl. z. B. US-PS 3 823 161). Sie zeigen außerdem eine sehr gute fungizide Wirksamkeit gegen Pilzarten, die beim Einsatz der Benzimidazolcarbamate Resistenzerscheinungen zeigen.

Ist $R^3$ Alkyl, so kann es geradkettig oder verzweigt sein und 1 bis 12, vorzugsweise 1 bis 6, insbesondere 1 bis 4 Kohlenstoffatome enthalten.
Beispielhaft seien Methyl, Ethyl, n-Propyl, i-Propyl, n-Butyl, i-Butyl, sec.-Butyl und tert.-Butyl genannt.

Bedeuten $R^1$ und $R^2$ Alkoxy, so können sie im Alkylteil geradkettig oder verzweigt sein und 1 bis 12, vorzugsweise 1 bis 6, insbesondere 1 bis 4 Kohlenstoffatome enthalten.
Beispielhaft seien Methoxy, Ethoxy, n-Propoxy, n-Butoxy, i-Butoxy, sec.-Butoxy und tert.-Butoxy genannt.

Bedeuten $R^1$ und $R^2$ Alkylthio, so können sie im Alkylteil geradkettig oder verzweigt sein und vorzugsweise 1 bis 6, insbesondere 1 bis 4 Kohlenstoffatome enthalten.
Beispielhaft seien Methyl-, Ethyl-, n-Propyl-, i-Propyl-, n-Butyl-, i-Butyl-, sec.-Butyl-und tert.-Butyl-thio genannt.

Bedeuten $R^1$ und $R^2$ Halogenalkoxy und Halogenalkylthio, so können sie im Alkylteil geradkettig oder verzweigt sein und 1 bis 6, vorzugsweise 1 bis 4, insbesondere 1 bis 2 Kohlenstoffatome und vorzugsweise 1 bis 6, insbesondere 1 bis 4 Halogenatome enthalten, wobei die Halogenatome gleich oder verschieden sein können und vorzugweise Fluor, Chlor oder Brom, insbesondere Fluor darstellen.
Beispielhaft seien genannt: Trifluormethoxy, Difluormethoxy, Tetrafluorethoxy, Chlortrifluorethoxy, Trifluormethylthio, Chlordifluormethylthio, Trifluorethylthio, Chlortrifluorethylthio und Tetrafluorethylthio.

$R^4$ enthält als Cycloalkylrest 3 bis 8, vorzugsweise 3 bis 6 Kohlenstoffatome.
Beispielhaft seien genannt: Cyclopropyl, Cyclobutyl, Cyclopentyl und Cyclohexyl.

Ist $R^4$ ein Alkenylrest, so kann er geradkettig oder verzweigt mit vorzugsweise 2 bis 6, insbesondere 2 bis 4 Kohlenstoffatomen sein.
Beispielhaft seien genannt: Ethenyl, Propen-1-yl, Propen-2-yl und Buten-3-yl.

Sind $R^1$, $R^2$ und $R^4$ gegebenenfalls substituierte Alkylreste oder sind $R^1$ und $R^2$ gegebenenfalls substituierte Alkylcarbonylreste, so können diese verzweigt oder unverzweigt sein und vorzugweise jeweils 1 bis 8, insbesondere 1 bis 6 und besonders bevorzugt 1 bis 4 Kohlenstoffatome enthalten.
Beispielhaft seien gegebenenfalls substituiertes Methyl, Ethyl, n-Propyl, iso-Propyl, n-Butyl, iso-Butyl, sec.-Butyl, tert.-Butyl, Methylcarbonyl, Ethylcarbonyl, n-Propylcarbonyl, iso-Propylcarbonyl, n-Butylcarbonyl, iso-Butyl carbonyl, sec.-Butylcarbonyl und tert.-Butylcarbonyl genannt.

Sind $R^1$, $R^2$ und $R^4$ gegebenenfalls substituierte Arylreste oder sind $R^1$ und $R^2$ gegebenenfalls substituierte Arylcarbonylreste, so enthalten die jeweiligen Arylteile vorzugsweise jeweils 6 bis 10 Kohlenstoffatome.
Beispielhaft seien gegebenenfalls substituiertes Naphthyl, Phenyl, Naphthylcarbonyl oder Phenylcarbonyl, insbesondere gegebenenfalls substituiertes Phenyl oder Phenylcarbonyl genannt.

Sind $R^1$ und $R^2$ gemeinsam mit den angrenzenden beiden Kohlenstoffatomen ein gegebenenfalls substituierter gesättiger oder ungesättiger carbocyclischer Ring, so handelt es sich um gesättigte oder ungesättigte 5-, 6-, 7-oder 8-gliedrige Ringe, vorzugsweise um gesättigte 5-, 6-, oder 7-gliedrige Ringe, wie insbesondere Cyclopentan, Cyclohexan und Cycloheptan.

X steht bevorzugt für Sauerstoff und

$R^3$ steht bevozugt für Wasserstoff.

Die gegebenenfalls substituierten Reste $R^1$, $R^2$, und $R^4$ können einen oder mehrere, vorzugsweise 1 bis 3, insbesondere 1 oder 2 gleiche oder verschiedene Substituenten tragen.

Als Substituenten für die Alkylreste $R^1$, $R^2$ und $R^4$ seien beispielhaft aufgeführt:
Halogen, wie Fluor, Chlor und Brom; Phenyl; Phenoxy; Phenylthio; Alkoxy mit vorzugsweise 1 bis 4, insbesondere 1 oder 2 Kohlenstoffatomen wie Methoxy, Ethoxy, n-und i-Propyloxy und n-, i-, sec.-und t-Butyloxy; Alkylthio mit vorzugsweise 1 bis 4, insbesondere 1 oder 2 Kohlenstoffatomen wie Methylthio, Ethylthio, n-und i-Propylthio und n-, i-, sec.-und t-Butylthio; Amino; Alkylamino und Dialkylamino mit jeweils vorzugsweise 1 bis 4, insbesondere 1 oder 2 Kohlenstoffatomen wie Methylamino, Ethylamino, n-Propylamino, i-Propylamino, n-Butylamino, i-Butylamino, Dimethylamino, Diethylamino, Di-n-propylamino, Di-i-propylamino, Di-n-butylamino und Di-i-Butylamino sowie Phenylamino.

Für die übrigen (nicht am Alkyl) Substitutionen kommen bei den Resten $R^1$, $R^2$ und $R^4$ noch in Frage:
Hydroxy, Cyano; Nitro; Alkyl mit vorzugsweise 1 bis 4, insbesondere 1 oder 2 Kohlenstoffatomen wie Methyl, Ethyl, n-und i-Propyl und n-, i-, sec.-und t-Butyl; Halogenalkyl mit vorzugsweise 1 bis 4, insbesondere 1 oder 2 Kohlenstoffatomen und vorzugsweise 1 bis 5, insbesondere 1 bis 3 Halogenatomen, wobei die Halogenatome gleich oder verschieden sind und als Halogenatome, vorzugsweise Fluor, Chlor oder Brom, insbesondere Fluor stehen wie Trifluormethyl; Halogenalkoxy mit vorzugsweise 1 bis 4, insbesondere 1 oder 2 Kohlenstoffatomen und vorzugsweise 1 bis 5, insbesondere 1 bis 3 Halogenatomen, wobei die Halogenatome gleich oder verschieden sind und als Halogenatome vorzugsweise Fluor, Chlor oder Brom, insbesondere Fluor stehen wie Trifluormethoxy; Halogenalkylthio mit vorzugsweise 1 bis 4, insbesondere 1 oder 2 Kohlenstoffatomen und vor zugsweise 1 bis 5, insbesondere 1 bis 3 Halogenatomen, wobei die Halogenatome gleich oder verschieden sind und als Halogenatome vorzugsweise Fluor, Chlor oder Brom, insbesondere Fluor stehen wie Trifluormethylthio; Alkylcarbonyl mit vorzugsweise 1 bis 4, insbesondere 1 oder 2 Kohlenstoffatomen wie Methylcarbonyl und Ethylcarbonyl; Alkoxyalkyl mit jeweils vorzugsweise 1 bis 4, insbesondere 1 oder 2 Kohlenstoffatomen im Alkylteil-bzw.Alkoxyteil, Methoxymethyl, Ethoxymethyl, Methoxyethyl und Ethoxyethyl; gegebenenfalls halogensubstituiertes Methylendioxy oder Ethylendioxy mit gegebenenfalls jeweils 1 bis 4 Halogenatomen, wobei als Halogenatome vorzugsweise Fluor, Chlor oder Brom, insbesondere Fluor und/oder Chlor stehen.

Halogen steht (wo nicht anders erläutert) für Fluor, Chlor, Brom und Iod, vorzugsweise für Fluor, Chlor und Brom.

Die erfindungsgemäß zu verwendenden substituierten Thienylamine sind durch die allgemeine Formel (I) allgemein definiert. Bevorzugt werden die Verbindungen der Formel (I) verwendet, wobei

X für Sauerstoff oder Schwefel steht,

$R^1$ und $R^2$ gleich oder verschieden sind und Wasserstoff; Halogen; Nitro; Cyano; Alkoxy mit 1 bis 6 Kohlenstoffatomen; Alkylthio mit 1 bis 6 Kohlenstoffatomen; gegebenenfalls einfach bis dreifach, gleich oder verschieden substituiertes Alkyl und Alkylcarbonyl mit jeweils 1 bis 6 Kohlenstoffatomen im Alkylteil bedeuten, wobei als Alkylsubstituenten in Frage kommen: Halogen, wie Fluor, Chlor und Brom; $C_1$-$C_4$-Alkoxy, $C_1$-$C_4$-Alkylthio, Phenyl, Phenoxy, Phenylthio, Amino, $C_1$-$C_4$-Alkylamino und Di-($C_1$-$C_4$)-alkylamino; oder gegebenenfalls einfach bis dreifach, gleich oder verschieden substituiertes Aryl und Arylcarbonyl mit jeweils 6 bis 10 Kohlenstoffatomen im Arylteil bedeuten, wobei als Arylsubstituenten in Frage kommen: Halogen, wie Fluor, Chlor, Brom, Cyano, Nitro, $C_1$-$C_4$-Alkyl, $C_1$-$C_4$-Alkoxy, $C_1$-$C_4$-Alkylthio, Phenyl, Phenoxy, Phenylthio, Amino, $C_1$-$C_4$-Alkylamino, Di-($C_1$-$C_4$)-alkylamino sowie Halogen-$C_1$-$C_4$-alkyl, Halogen-$C_1$-$C_4$-alkoxy und Halogen-$C_1$-$C_4$-alkylthio mit jeweils bis zu 5 Halogenatomen, ferner $C_1$-$C_4$-Alkylcarbonyl, $C_1$-$C_4$-Alkoxy-$C_1$-$C_4$-alkyl und gegebenenfalls halogensubstituiertes Methylendioxy oder Ethylendioxy mit gegenbenenfalls 1 bis 4 Halogenatomen, oder

$R^1$ und $R^2$ gemeinsam mit den angrenzenden beiden Kohlenstoffatomen für einen gegebenenfalls durch $C_1$-$C_6$-Alkyl substituierten gesättigten oder ungesättigten 5-, 6-, 7-oder 8-gliedrigen carbocyclischen Ring stehen,

$R^3$ für Wasserstoff oder $C_1$-$C_6$-Alkyl steht und

$R^4$ gegebenenfalls durch Halogen, $C_1$-$C_4$-Alkoxy, $C_1$-$C_4$-Alkylthio, Phenyl, Phenoxy, Phenylthio, Amino, $C_1$-$C_4$-Alkylamino oder Di-$C_1$-$C_4$-alkylamino substituiertes Alkyl mit 1 bis 6 Kohlenstoffatomen, Cycloalkyl mit 3 bis 8 Kohlenstoffatomen, Alkenyl mit 2 bis 6 Kohlenstoffatomen oder gegebenenfalls einfach bis dreifach, gleich oder verschieden substituiertes Phenyl und Naphthyl bedeutet, wobei als Phenylsubstituenten in Frage kommen:
Halogen, Cyano, $C_1$-$C_4$-Alkyl, $C_1$-$C_4$-Alkoxy, $C_1$-$C_4$-Alkylthio, Phenyl, Phenoxy, Phenylthio, Amino, $C_1$-$C_4$-Alkylamino, Di-$C_1$-$C_4$-alkylamino, $C_1$-$C_4$-Alkoxy-$C_1$-$C_4$-alkyl, Halogen-$C_1$-$C_4$-alkyl, Halogen-$C_1$-$C_4$-alkoxy, Halogen-$C_1$-$C_4$-alkylthio und gegebenenfalls durch Fluor und/ oder Chlor substituiertes Methylendioxy oder Ethylendioxy.

Besonders bevorzugt werden die Verbindungen der allgemeinen Formel (I) verwendet, in denen

X für Sauerstoff steht,

$R^1$ und $R^2$ gleich oder verschieden sind und für Wasserstoff, Nitro, gegebenenfalls durch Fluor, Chlor oder Brom substituiertes $C_1$-$C_6$-Alkyl, für gegebenenfalls durch $C_1$-$C_4$-Alkyl, Fluor, Chlor, Brom, Halogen-$C_1$-$C_2$-alkyl und/oder Halogen-$C_1$-$C_2$-alkoxy substituiertes Phenyl, für Benzoyl oder für $C_1$-$C_2$-Alkoxycarbonyl stehen, oder

$R^1$ und $R^2$ gemeinsam mit den angrenzenden beiden Kohlenstoffatomen für einen gegebenenfalls durch $C_1$-$C_4$-Alkyl substituierten, gesättigten 5-, 6-, 7-oder 8-gliedrigen carbocyclischen Ring stehen,

$R^3$ für Wasserstoff oder $C_1$-$C_4$-Alkyl steht,

$R^4$ für $C_1$-$C_6$-Alkyl, $C_1$-$C_4$-Alkylthio-$C_1$-$C_4$-alkyl, $C_3$-$C_8$-Cycloalkyl, $C_2$-$C_4$-Alkenyl, für gegebenenfalls durch $C_1$-$C_4$-Alkyl, $C_1$-$C_4$-Alkoxy, Halogen-$C_1$-$C_2$-alkyl, Nitro und/oder Chlor substituiertes Phenyl und für Naphthyl steht.

Ganz besonder bevorzugt werden diejenigen Verbindungen der allgemeinen Formel (I) verwendet, in denen

X für Sauerstoff steht,

$R^1$ und $R^2$ gleich oder verschieden sind und für Wasserstoff, Nitro, Methyl, Ethyl, n-Propyl, n-Butyl, i-Butyl,

sec.-Butyl, tert,-Butyl, n-Pentyl, Phenyl, Benzoyl oder Methylcarbonyl stehen, oder

$R^1$ und $R^2$ gemeinsam mit den angrenzenden beiden Kohlenstoffatomen für gegebenenfalls durch Methyl und/oder Ethyl substituiertes Cyclopentan, Cyclohexan oder Cycloheptan stehen,

$R^3$ für Wasserstoff steht und

$R^4$ für Methyl, Ethyl, n-Propyl, i-Propyl, n-Butyl, i-Butyl, sec.-Butyl, tert.-Butyl, n-Pentyl, Cyclopropyl, Cyclopentyl, Cyclohexyl, für gegebenenfalls durch Chlor, Nitro, Methyl, Ethyl, n-Propyl, i-Pro pyl, Methoxy und/oder Trifluormethyl substituiertes Phenyl oder Naphthyl steht.

Die erfindungsgemäß zu verwendenden Wirkstoffe der allgemeinen Formel (I) sind bekannt und/oder lassen sich in einfacher Weise nach bekannten Methoden herstellen (vergl. z. B. EP-OS 4 931 und US-PS 4 317 915). So lassen sich z. B. 2-Amino-thiophen-Derivate mit (1) (Thio)Phosgen und Alkoholen oder (2) mit Halogen(thio)ameisensäureestern gemäß dem folgenden Formelschema umsetzen.

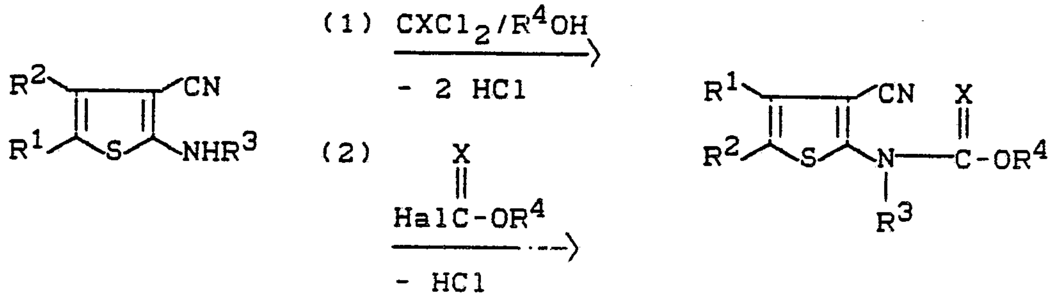


Die Reaktion wird üblicherweise bei Normaldruck und einer Temperatur von -20 °C bis +40 °C durchgeführt, gegebenenfalls in Anwesenheit einer Hilfsbase, wie z. B. Triethylamin und in Gegenwart inerter Verdünnungsmittel, wie z. B. Toluol oder Chloroform.

Die erfindungsgemäß zu verwendenden Wirkstoffe weisen eine starke fungizide Wirkung auf. Sie werden bevorzugt zur Bekämpfung von unerwünschten Pilzen, die eine Resistenz gegen Benzimidazolcarbamate zeigen, eingesetzt. Bevorzugt werden sie eingegetzt bei der Bekämpfung von Grauschimmelpilzen, wie z. B. Botrytis cinerea an Gartenbohnen und Getreidekrankheiten, wie z. B. Cercosporella herpotrichoides (Halmbruch).

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Erkrankungen, die unter die oben aufgezählten Oberbegriffe fallen, genannt:

Pythium-Arten, wie beispielsweise Pythium ultimum;
Phytophthora-Arten, wie biespielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopora viticola;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea
(Konidienform: Drechslera, Syn: Helminthosporium);
Cochliobolus-Arten, wie beispielsweise Cochliobolus sativus;
(Konidienform: Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;

Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz-und Saatgut, und des Bodens.

Die Wirkstoffe können in die üblichen Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Träger stoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier-und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycol-ether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo-und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäß zu verwendenden Wirkstoffe können in den Formulierungen in Mischung mit anderen bekannten Wirk stoffen vorliegen, wie Fungizide, Insektizide, Akarizide und Herbizide, sowie in Mischungen mit Düngemitteln und Wachstumsregulatoren.

Die erfindungsgemäß zu verwendenden Wirkstoffe können als solche, in Form ihrer Formulierungen oder den daraus bereiteten Anwendungsformen, wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0.01 bis 10 g, benötigt.

Bei Behandlung des Bodens sind Wirkstoffkonzentrationen von 0,00001 bis 0,1 Gew.-%, vorzugsweise von 0.0001 bis 0.02 %, am Wirkungsort erforderlich.

Herstellungsbeispiele

Beispiel 1

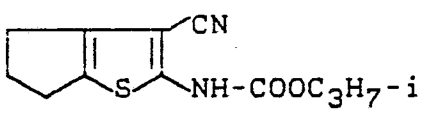

Zu einer Lösung von 7,2 g (0,044 Mol) 2-Amino-3-cyano-4,5-trimethylen-thiophen und 9,8 g (0,097 Mol) Triethylamin in 200 ml abs. Chloroform werden bei -10 °C 36 ml einer 1,93 molaren Lösung von Phosgen in Toluol (ca. 0,069 Mol Phosgen) gegeben und 15 Min. ohne Kühlung nachgerührt. Anschließend werden 2,9 g (0,048 Mol) Isopropanol zugetropft, eine Stunde nachgerührt und auf 700 ml Wasser gegossen. Die organische Phase wird abgetrennt, mit 5 %iger Natriumhydrogenphosphatlösung und Wasser gewaschen, getrocknet und eingeengt. Der Rückstand wird aus Chloroform/Petrolether umkristallisiert.

Man erhält 7,1 g (65 % der Theorie) 3-Cyano-2-iso-propoxycarbonylamino-4,5-trimethylenthiophen vom Schmelzpunkt 120 °C.

Beispiel 2

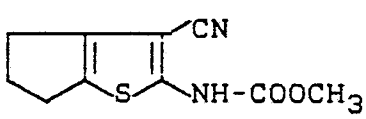

Eine Mischung aus 8,2 g (0,05 Mol) 2-Amino-3-cyano-4 5-trimethylen-thiophen, 4,7 g (0.05 Mol) Chlorameisensäure methylester und 3,95 g (0,05 Mol) Pyridin in 100 ml absolutem Chloroform wird 6 Stunden bei 20 °C gerührt. Anschließend wird mit Wasser, 10 %iger Salzsäure und nochmals mit Wasser gewaschen, über Natriumsulfat getrocknet und eingeengt. Das Rohprodukt wird aus Chloroform/Hexan umkristallisiert.

Man erhält 9,2 g (83 % der Theorie) 3-Cyano-2-methoxycarbonylamino-4,5-trimethylen-thiophen vom Schmelzpunkt 164 °C.

Analog Beispiel (1) oder (2) können die folgenden Verbindungen der Formel (I) hergestellt werden:

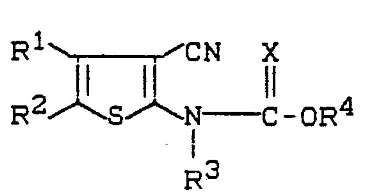

(I)

Tabelle 1

| Beisp.-Nr. | $R^1$ | $R^2$ | $R^3$ | $R^4$ | X | Fp /[°C] |
|---|---|---|---|---|---|---|
| 3 | | $-(CH_2)_5-$ | H | $-CH_3$ | O | 129 |
| 4 | | $-(CH_2)_4-$ | H | $-CH_3$ | O | 157 |
| 5 | | $-(CH_2)_3-$ | H | $-C_4H_9$-tert. | O | 148 |
| 6 | | $-(CH_2)_3-$ | H | 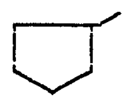 | O | 140 |
| 7 | | $-(CH_2)_3-$ | H | 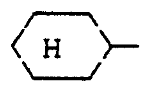 | O | 141 |
| 8 | | $-(CH_2)_3-$ | H | 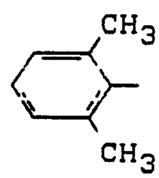 | O | >230 |
| 9 | | $-(CH_2)_3-$ | H | 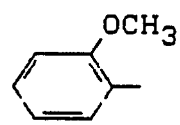 | O | 194 |
| 10 | | $-(CH_2)_5-$ | H | $-C_3H_7$-i | O | 112 |
| 11 | | $-(CH_2)_5-$ | H | $-C_4H_9$-tert. | O | 152 |
| 12 | | $-(CH_2)_5-$ | H | $-C_4H_9$-sec. | O | >220 |
| 13 | | $-(CH_2)_5-$ | H | 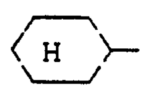 | O | 124 |
| 14 | | $-(CH_2)_5-$ | H | 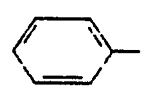 | O | 142 |

<u>Tabelle 1</u> - Fortsetzung

| Beisp.-Nr. | $R^1$ | $R^2$ | $R^3$ | $R^4$ | X | Fp /[°C] |
|---|---|---|---|---|---|---|
| 15 | | $-(CH_2)_5-$ | H | 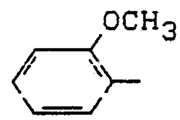 | O | 139 |
| 16 | | $-(CH_2)_5-$ | H | 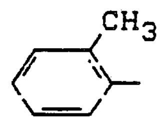 | O | 165 |
| 17 | | $-(CH_2)_5-$ | H | 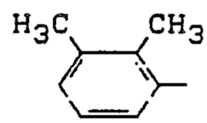 | O | 192 |
| 18 | | $-(CH_2)_5-$ | H | 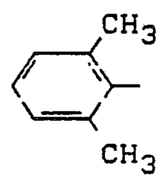 | O | 188 |
| 19 | | $-(CH_2)_5-$ | H | 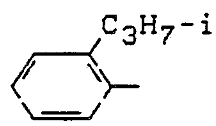 | O | 127 |
| 20 | | $-(CH_2)_5-$ | H | 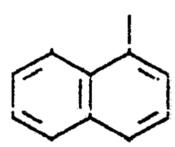 | O | 112 |
| 21 | | $-(CH_2)_4-$ | H | $-C_3H_7$-i | O | >220 |
| 22 | | $-(CH_2)_4-$ | H | 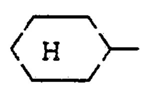 | O | 186 |
| 23 | | $-(CH_2)_4-$ | H | 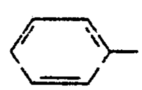 | O | >250 |

Tabelle 1 - Fortsetzung

| Beisp.-Nr. | $R^1$ | $R^2$ | $R^3$ | $R^4$ | X | Fp /[°C] |
|---|---|---|---|---|---|---|
| 24 | $-(CH_2)_4-$ | | H | 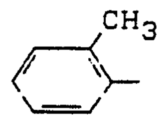 | O | 199 |
| 25 | $-(CH_2)_4-$ | | H | 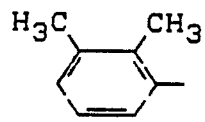 | O | 224 |
| 26 | $-(CH_2)_4-$ | | H | 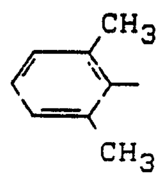 | O | 200 |
| 27 | $-(CH_2)_3-$ | | H | $-C_2H_5$ | O | 148 |
| 28 | $-(CH_2)_4-$ | | H | $-C_2H_5$ | O | 92 |
| 29 | $-(CH_2)_5-$ | | H | $-C_2H_5$ | O | 133 |
| 30 | H | $-CH_3$ | H | $-CH_3$ | O | 187 |
| 31 | H | $-CH_3$ | H | $-C_2H_5$ | O | 94 |
| 32 | $-CH_3$ | $-CH_3$ | H | $-CH_3$ | O | 154 |
| 33 | $-CH_3$ | $-CH_3$ | H | $-C_2H_5$ | O | 135 |
| 34 | $-CH_3$ | H | H | $-CH_3$ | O | 154 |
| 35 | $-CH_3$ | H | H | $-C_2H_5$ | O | 125 |
| 36 | H | H | H | $-CH_3$ | O | 166 |
| 37 | H | H | H | $-C_2H_5$ | O | 102 |
| 38 | $-(CH_2)_3-$ | | H | $-C_4H_9$-i | O | 141 |
| 39 | $-(CH_2)_4-$ | | H | $-C_4H_9$-i | O | 127 |

Tabelle 1 - Fortsetzung

| Beisp.-Nr. | $R^1$ | $R^2$ | $R^3$ | $R^4$ | X | Fp /[° C] |
|---|---|---|---|---|---|---|
| 40 | $-(CH_2)_5-$ | | H | $-C_4H_9-i$ | O | 139 |
| 41 | $-CH_3$ | H | H | $-C_4H_9-i$ | O | 105 |
| 42 | H | $-CH_3$ | H | $-C_4H_9-i$ | O | 65 |
| 43 | $-CH_3$ | $-CH_3$ | H | $-C_4H_9-i$ | O | 138 |
| 44 | H | H | H | $-C_4H_9-i$ | O | 95 |
| 45 | $-C_2H_5$ | $-CH_3$ | H | $-C_4H_9-i$ | O | 106 |
| 46 | $-C_2H_5$ | $-CH_3$ | H | $-CH_3$ | O | 123 |
| 47 | $-CH_2CHCH_2C(CH_3)_2-$<br>\| <br>$CH_3$ | | H | $-CH_3$ | O | 116 |
| 48 | $-CH_2CHCH_2C(CH_3)_2-$<br>\| <br>$CH_3$ | | H | $C_4H_9-i$ | O | 117 |
| 49 | $-CH_3$ | -H | H | $-C_3H_7-i$ | O | 87 |
| 50 | H | $-CH_3$ | H | $-C_3H_7-i$ | O | 92 |
| 51 | H | H | H | $-C_3H_7-i$ | O | 83 |
| 52 | $-CH_3$ | $-CH_3$ | H | $-C_3H_7-i$ | O | 100 |
| 53 | $-C_2H_5$ | $-CH_3$ | H | $-C_3H_7-i$ | O | 93 |
| 54 | $-CH(CH_2)_3-$<br>\| <br>$CH_3$ | | H | $-C_3H_7-i$ | O | 86 |
| 55 | $-CH_2CH(CH_2)_2-$<br>\| <br>$CH_2$ | | H | $-C_3H_7-i$ | O | 135 |

Tabelle 1 - Fortsetzung

| Beisp.-Nr. | $R^1$ | $R^2$ | $R^3$ | $R^4$ | X | Fp /[°C] |
|---|---|---|---|---|---|---|
| 56 | $-CH_2CH(CH_2)_2-$<br>$CH_3$ | | H | $-CH_3$ | O | 168 |
| 57 | $-CH_2CH(CH_2)_2-$<br>$CH_3$ | | H | $-C_4H_9-i$ | O | 146 |
| 58 | $-(CH_2)_2CHCH_2-$<br>$CH_3$ | | H | $-C_3H_7-i$ | O | 146 |
| 59 | $-(CH_2)_2CHCH_2-$<br>$CH_3$ | | H | $-CH_3$ | O | 161 |
| 60 | $-(CH_2)_2CHCH_2-$<br>$CH_3$ | | H | $-C_4H_9-i$ | O | 143 |
| 61 | $-(CH_2)_2CH-$<br>$CH_3$ | | H | $-CH_3$ | O | 130 |
| 62 | $-(CH_2)_2CH-$<br>$CH_3$ | | H | $-C_3H_7-i$ | O | 113 |
| 63 | $-C_2H_5$ | $-CH_3$ | H | 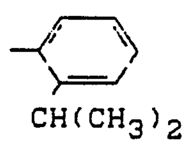 | O | 159 |
| 64 | $-CH_2CH(CH_2)_2-$<br>$CH_3$ | | H | 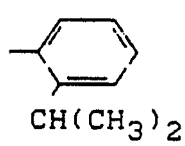 | O | 194 |
| 65 | $-(CH_2)_2CHCH_2-$<br>$CH_3$ | | H | 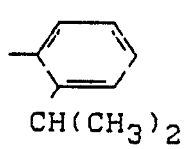 | O | 137 |

Tabelle 1 - Fortsetzung

| Beisp.-Nr. | $R^1$ | $R^2$ | $R^3$ | $R^4$ | X | Fp /[°C] |
|---|---|---|---|---|---|---|
| 66 | H | H | H | 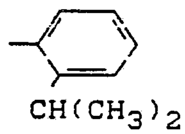 | O | 99-101 |
| 67 | $-(CH_2)_4-$ | | H | $-C_4H_9$-sec. | O | 103 |
| 68 | H | H | H | $-C_4H_9$-sec. | O | Öl |
| 69 | H | $-CH_3$ | H | $-C_4H_9$-sec. | O | 48 |

Beispiel A

Botrytis-Test (Bohne) protektiv (BCM-resistenter Botrytis-Stamm)
Lösungsmittel: 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden auf jedes Blatt 2 kleine mit Botrytis cinerea bewachsene Agarstückchen aufgelegt. Die inokulierten Pflanzen werden in einer abgedunkelten, feuchten Kammer bei 20°C aufgestellt. 3 Tage nach der Inokulation wird die Größe der Befallsflecken auf den Blättern ausgewertet.

Die erfindungsgemäß eingesetzten Verbindungen zeigen in diesem Test eine deutliche Überlegenheit in der Wirksamkeit gegenüber dem Stand der Technik.

Tabelle A

Botrytis-Test (Bohne)/protektiv
(BCM-resistenter Botrytis-Stamm)

| Wirkstoff | Befall in % bei einer Wirkstoffkonzentration von 50 ppm |
|---|---|
| 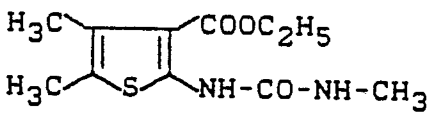 (Bekannt) (A) | 49 |
| 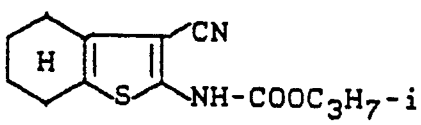 (21) | 21 |

Beispiel B

Agarplattentest

Verwendeter Nährboden
39 Gewichtsteile Potato-Dextrose-Agar
20 Gewichtsteile Agar-Agar
werden in 1000 ml destilliertem Wasser gelöst und 20 Minuten bei 121 °C autoklaviert.
Lösungsmittel: 50 Gewichtsteile Aceton
950 Gewichtsteile Wasser
Mengenverhältnis von Lösungsmittel zu Nährboden: 1 : 100

Zur Herstellung einer zweckmäßigen Wirkstoffzubereitung vermischt man 1 Gewichtsteil Wirkstoff mit der angegebenen Menge Lösungsmittel.

Das Konzentrat wird im genannten Mengenverhältnis mit dem flüssigen Nährboden gründlich vermischt und in Petrischalen gegossen.

Ist der Nährboden erkaltet und fest, werden die Platten mit folgenden Mikroorganismen beimpft und bei ca. 21 °C inkubiert: Pseudocercosporella herpotrichoides (BCM-resistenter Stamm)

Die Auswertung erfolgt nach 10 Tagen, wobei dei Wachstumshemmung als Maß für die Wirkung der Präparate herangezogen wird.

Die erfindungsgemäß eingesetzten Verbindungen zeigen in diesem Test eine deutliche Überlegenheit gegenüber dem Stand der Technik.

**Tabelle B**

**Agarplatten-Test**

Pseudocercosporella herpotrichoides
(BCM-resistenter Stamm)

| Wirkstoff | Wirkstoffkonzentration in ppm | Myzelzuwachs/ Durchmesser in mm |
|---|---|---|
| unbehandelt | - | 13,5 |
| 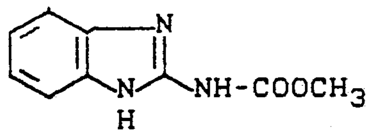 (Bekannt) (B) | 10 | 13,5 |
| 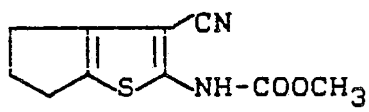 (2) | 10 | 6,5 |
| 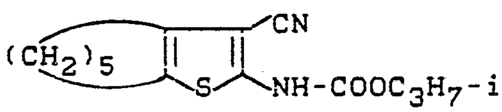 (1) | 10 | 10,8 |
| 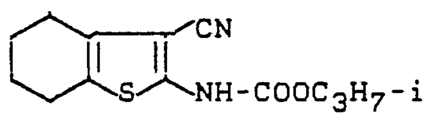 (21) | 10 | 2,5 |
| 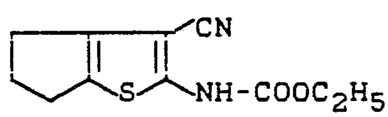 (27) | 10 | 8,0 |
| 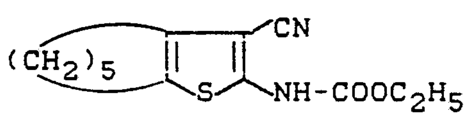 (29) | 10 | 9,0 |
| 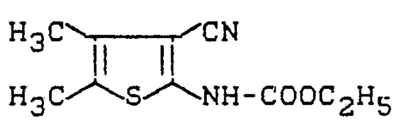 (33) | 10 | 1,8 |

**Ansprüche**

1. Fungizide Mittel, gekennzeichnet durch einen Gehalt an mindestens einem substituierten Thienylamin der allgemeinen Formel (I)

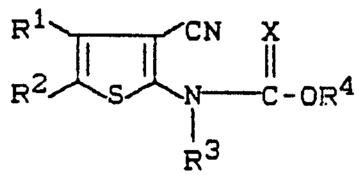

(I)

in welcher

X für Sauerstoff oder Schwefel steht,

$R^1$ und $R^2$ gleich oder verschieden sind und für Wasserstoff, Halogen, Nitro, Cyano, Alkoxy, Alkylthio, Halogenalkoxy, Halogenalkylthio oder für gegebenenfalls substituierte Reste aus der Reihe Alkyl, Aryl, Alkylcarbonyl oder Arylcarbonyl stehen, oder

$R^1$ und $R^2$ gemeinsam mit den angrenzenden beiden Kohlenstoffatomen für einen gegebenenfalls durch Alkyl substituierten gesättigten oder ungesättigten carbocyclischen Ring stehen,

$R^3$ für Wasserstoff oder Alkyl steht und

$R^4$ für gegebenenfalls substituiertes Alkyl, für Cycloalkyl, Alkenyl oder für gegebenenfalls substituiertes Aryl steht.

2. Fungizides Mittel gemäß Anspruch 1, dadurch gekennzeichnet, daß in der allgemeinen Formel (I)

X für Sauerstoff oder Schwefel steht,

$R^1$ und $R^2$ gleich oder verschieden sind und Wasserstoff; Halogen; Nitro; Cyano; Alkoxy mit 1 bis 6 Kohlenstoffatomen; Alkylthio mit 1 bis 6 Kohlenstoffatomen; gegebenenfalls einfach bis dreifach, gleich oder verschieden substituiertes Alkyl und Alkylcarbonyl mit jeweils 1 bis 6 Kohlenstoffatomen im Alkylteil bedeuten, mit Substituenten im Alkylteil aus der Reihe Halogen, $C_1$-$C_4$-Alkoxy, $C_1$-$C_4$-Alkylthio, Phenyl, Phenoxy, Phenylthio, Amino, $C_1$-$C_4$-Alkylamino und Di-($C_1$-$C_4$-)-alkylamino; oder gegebenenfalls einfach bis dreifach, gleich oder verschieden substituiertes Aryl und Arylcarbonyl mit jeweils 6 bis 10 Kohlenstoffatomen im Arylteil bedeuten, mit Arylsubstituenten aus der Reihe Halogen, Cyano, Nitro, $C_1$-$C_4$-Alkyl, $C_1$-$C_4$-Alkoxy, $C_1$-$C_4$-Alkylthio, Phenyl, Phenoxy, Phenylthio, Amino, $C_1$-$C_4$-Alkylamino, Di-($C_1$-$C_4$)-alkylamino sowie Halogen-$C_1$-$C_4$-alkyl, Halogen-$C_1$-$C_4$-alkoxy und Halogen-$C_1$-$C_4$-alkylthio mit jeweils bis zu 5 Halogenatomen, $C_1$-$C_4$-Alkylcarbonyl, $C_1$-$C_4$-Alkoxy-$C_1$-$C_4$-alkyl und gegebenenfalls halogensub stituiertes Methylendioxy oder Ethylendioxy mit gegebenenfalls 1 bis 4 Halogenatomen, oder

$R^1$ und $R^2$ gemeinsam mit den angrenzenden beiden Kohlenstoffatomen für einen gegebenenfalls durch $C_1$-$C_6$-Alkyl substituierten gesättigten oder ungesättigten 5-, 6-, 7-oder 8-gliedrigen carbocyclischen Ring stehen,

$R^3$ für Wasserstoff oder $C_1$-$C_6$-Alkyl steht und

$R^4$ gegebenenfalls durch Halogen, $C_1$-$C_4$-Alkoxy, $C_1$-$C_4$-Alkylthio, Phenyl, Phenoxy, Phenylthio, Amino, $C_1$-$C_4$-Alkylamino oder Di-$C_1$-$C_4$-alkylamino substituiertes Alkyl mit 1 bis 6 Kohlenstoffatomen, Cycloalkyl mit 3 bis 8 Kohlenstoffatomen, Alkenyl mit 2 bis 6 Kohlenstoffatomen oder gegebenenfalls einfach bis dreifach, gleich oder verschieden substituiertes Phenyl und Naphthyl bedeutet, mit Phenylsubstituenten aus der Reihe Halogen, Cyano, $C_1$-$C_4$-Alkyl, $C_1$-$C_4$-Alkoxy, $C_1$-$C_4$-Alkylthio, Phenyl, Phenoxy, Phenylthio, Amino, $C_1$-$C_4$-Alkylamino, Di-$C_1$-$C_4$-alkylamino, $C_1$-$C_4$-Alkoxy-$C_1$-$C_4$-alkyl, Halogen-$C_1$-$C_4$-alkyl, Halogen-$C_1$-$C_4$-alkoxy, Halogen-$C_1$-$C_4$-alkylthio und gegebenenfalls durch Fluor und/oder Chlor substituiertes Methylendioxy oder Ethylendioxy.

3. Fungizides Mittel gemäß Anspruch 1, dadurch gekennzeichnet, daß in der allgemeinen Formel (I)

X für Sauerstoff steht,

$R^1$ und $R^2$ gleich oder verschieden sind und für Wasserstoff, Nitro, gegebenenfalls durch Fluor, Chlor oder Brom substituiertes $C_1$-$C_6$-Alkyl, für gegebenenfalls durch $C_1$-$C_4$-Alkyl, Fluor, Chlor, Brom, Halogen-$C_1$-$C_2$-alkyl und/oder Halogen-$C_1$-$C_2$-alkoxy substituiertes Phenyl, für Benzoyl oder für $C_1$-$C_2$-Alkoxycarbonyl stehen, oder

$R^1$ und $R^2$ gemeinsam mit den angrenzenden beiden Kohlenstoffatomen für einen gegebenenfalls durch $C_1$-$C_4$-Alkyl substituierten, gesättigten 5-, 6-, 7-oder 8-gliedrigen carbocyclischen Ring stehen,

$R^3$ für Wasserstoff oder $C_1$-$C_4$-Alkyl steht,

$R^4$ für $C_1$-$C_6$-Alkyl, $C_1$-$C_4$-Alkylthio-$C_1$-$C_4$-alkyl, $C_3$-$C_8$-Cycloalkyl, $C_2$-$C_4$-Alkenyl, für gegebenenfalls durch $C_1$-$C_4$-Alkyl, $C_1$-$C_4$-Alkoxy, Halogen-$C_1$-$C_2$-alkyl, Nitro und/oder Chlor substituiertes Phenyl und für Naphthyl steht.

4. Fungizides Mittel gemäß Anspruch 1, dadurch gekennzeichnet, daß in der allgemeinen Formel (I)

X für Sauerstoff steht,

$R^1$ und $R^2$ gleich oderverschieden sind und für Wasserstoff, Nitro, Methyl, Ethyl, n-Propyl, n-Butyl, i-Butyl, sec.-Butyl, tert.-Butyl, n-Pentyl, Phenyl, Benzol oder Methylcarbonyl stehen, oder

$R^1$ und $R^2$ gemeinsam mit den angrenzenden beiden Kohlenstoffatomen für gegebenenfalls durch Methyl und/oder Ethyl substituiertes Cyclopentan, Cyclohexan oder Cycloheptan stehen,

$R^3$ für Wasserstoff steht und

$R^4$ für Methyl, Ethyl, n-Propyl, i-Propyl, n-Butyl, i-Butyl, sec.-Butyl, tert.-Butyl, n-Pentyl, Cyclopropyl, Cyclopentyl, Cyclohexyl, für gegebenenfalls durch Chlor, Nitro, Methyl, Ethyl, n-Propyl, i-Propyl, Methoxy und/oder Trifluormethyl substituiertes Phenyl oder Naphthyl steht.

5. Verfahren zur Bekämpfung von pilzlichen Schädlingen, dadurch gekennzeichnet, daß man substituierte Thienylamine der allgemeinen Formel (I) gemäß Anspruch 1 auf pilzliche Schädlinge und/oder ihren Lebensraum einwirken läßt.

6. Verwendung von substituierten Thienylaminen der allgemeinen Formel (I) gemäß Anspruch 1 zur Bekämpfung von pilzlichen Schädlingen.

7. Verfahren zur Herstellung von fungiziden Mitteln, dadurch gekennzeichnet, daß man substituierte Thienylamine der allgemeinen Formel (I) gemäß Anspruch 1 mit Streckmitteln und/oder oberflächenaktiven Mitteln mischt.